# EUROPEAN PATENT APPLICATION

(11) **EP 1 449 848 A1**
(43) Date of publication of application: **25.08.2004**
(21) Application number: 04003752.5
(22) Date of filing: 19.02.2004
(51) Int. Cl.: C07K 1/113, C07K 14/51, C07K 14/475, C12N 15/70, C12P 21/02

(54) **Method for the production of cystine-knot proteins**

(30) Priority: 20.02.2003 EP 03003219
(71) Applicant: GBF German Research Centre for Biotechnology, 38124 Braunschweig (DE)
(72) Inventor: Vallejo, Luis Felipe, 38124 Braunschweig (DE); Rinas, Ursula, 38118 Braunschweig (DE)
(74) Representative: VOSSIUS & PARTNER

(57) **Abstract**

The present invention relates to a method of producing a biologically active recombinant cystine-knot protein comprising (a) solubilisation of inclusion bodies comprising said cystine-knot protein produced in a bacterium in the presence of a chaotropic agent; (b) renaturation of the solubilized cystine-knot protein in batch or by pulse addition of said solubilized cystine-knot protein to a refolding buffer essentially devoid of NaCl having a pH value of 7.5 to 9.5 and preferably of 8 to 9 comprising (ba) an aggregation suppressor in a final concentration of at least 0.5 mol/L; (bb) a mixture of reduced and oxidized glutathione wherein the ratio of reduced to oxidized glutathione is equal or above 1:10; and (bc) a solubilizing chaotropic agent in a non-denaturing concentration, wherein said renaturation is carried out at a temperature between 0°C and 20°C; (c) dialysis of the renatured cystine-knot protein against or dilution of the renatured cystine-knot protein into a buffer having an alkaline pH value comprising a solubilising non-ionic agent in a non-denaturing concentration; (d) binding to a heparin matrix of the product obtained in step (c) at a conductivity between 15 and 25 mS/cm; and (e) elution of the renatured biologically active cystine-knot protein.

## Description

The present invention relates to a method of producing a biologically active recombinant cystine-knot protein comprising (a) solubilisation of inclusion bodies comprising said cystine-knot protein produced in a bacterium in the presence of a chaotropic agent; (b) renaturation of the solubilized cystine-knot protein in batch or by pulse addition of said solubilized cystine-knot protein to a refolding buffer essentially devoid of NaCl having a pH value of 7.5 to 9.5 and preferably of 8 to 9 comprising (ba) an aggregation suppressor in a final concentration of at least 0.5 mol/L; (bb) a mixture of reduced and oxidized glutathione wherein the ratio of reduced to oxidized glutathione is equal or above 1:10; and (bc) a solubilizing chaotropic agent in a non-denaturing concentration, wherein said renaturation is carried out at a temperature between 0°C and 20°C; (c) dialysis of the renatured cystine-knot protein against or dilution of the renatured cystine-knot protein into a buffer having an alkaline pH value comprising a solubilising non-ionic agent in a non-denaturing concentration; (d) binding to a heparin matrix of the product obtained in step (c) at a conductivity between 15 and 25 mS/cm; and (e) elution of the renatured biologically active cystine-knot protein.

In this specification, a number of documents is cited. The disclosure content of these documents, including manufacturers' manuals, is herewith incorporated by reference.

Bone morphogenetic protein-2 (BMP-2) plays an important role in the development of many organs and tissues (#3558). The most important feature of BMP-2 is its ability to induce bone formation, thus rendering it to a protein of eminent pharmaceutical importance (#3593). It has been shown many times that it can accelerate the healing process of broken bones and other bone related diseases (*e.g.* #3559; #3560; #3561). Moreover, the safeness of BMP-2 containing implants has been reported in various cases (*e.g.* #3559; #3560; #3562) and, more recently, the FDA has approved the first recombinant human BMP-2 containing product aimed for promoting the healing process of certain types of spinal degenerative disc diseases (Infuse™, Medtronic, USA)
BMP-2 is a member of the transforming growth factor-β family. It is a disulfide-bonded dimer where the two subunits are assembled head-to-tail and connected by one disulfide bond. The monomer carries a characteristic arrangement of three intertwined disulfide bridges known as a cystine knot (#2859). The cystine knot scaffold stabilizes the monomer that lacks a hydrophobic core common to globular proteins (#2859). During dimerization a hydrophobic core is formed among the two monomers, however, large portions of the dimer surface are still very hydrophobic, thereby causing low solubility of BMP-2 in aqueous solutions (#2859; #2861).
Recombinant human BMP-2 has been produced with various expression systems including production as active protein in mammalian cell cultures (#3594; #3595) or, alternatively, in form of inclusion bodies in bacteria such as *Escherichia coli* (#2861). It has been shown that bone inducing properties of rhBMP-2 generated by refolding from *E. coli* produced inclusion bodies are comparable to those of the mammalian cell-derived product (#3591). As large quantities of rhBMP-2 are required for further development and clinical applications an efficient recombinant production strategy is a necessity.
The highest yield reported so far has been obtained with recombinant *E. coli* where rhBMP-2 is produced in form of cytoplasmic inclusion bodies (#3233). Recovery of biologically active rhBMP-2 can be achieved when refolding of solubilized inclusion body protein is carried out in the presence of non-detergent zwitterionic aggregation suppressors such as acid substituted pyridines or aminocyclohexanes (#3233; #3085).

Vallejo et al., J. Biotechnol. 94 (2002), 185-194 have demonstrated that biologically active bone morphogenetic protein-2 (BMP-2) can be purified to a high degree from inclusion bodies after overexpression in E. coli. The process employed solubilisation using a buffer containing guanidinium hydrochloride, renaturation in the presence of the oxidized and reduced forms of glutathione and 2-(cyclohexylamino)ethansulfonic acid (CHES), followed by dialysis against a urea containing buffer and affinity chromatography using a heparin column. Whereas significant yields of BMP-2 could be recovered by this process, those yields still do not appear appropriate as a basis for the establishment of a highly effective large scale production process for cystine-knot proteins. The technical problem underlying the present invention was therefore to develop a technology that may form the basis for such a highly effective large scale production process for cystine-knot proteins. Such a process should in particular yield high absolute amounts of biologically active proteins as assessed from the input cell mass.

The solution to said technical problem is achieved by providing the embodiments characterized by the claims.

Accordingly, the present invention relates to a method of producing a biologically active recombinant cystine-knot protein comprising (a) solubilisation of inclusion bodies comprising said cystine-knot protein produced in a bacterium in the presence of a chaotropic agent; (b) renaturation of the solubilized cystine-knot protein in batch or by pulse addition of said solubilized cystine-knot protein to a refolding buffer essentially devoid of NaCl having a pH value of 7.5 to 9.5 and preferably of 8 to 9 comprising (ba) an aggregation suppressor in a final concentration of at least 0.5 mol/L; (bb) a mixture of reduced and oxidized glutathione wherein the ratio of reduced to oxidized glutathione is equal or above 1:10; and (bc) a solubilizing chaotropic agent in a non-denaturing concentration, wherein said renaturation is carried out at a temperature between 0°C and 20°C; (c) dialysis of the renatured cystine-knot protein against or dilution of the renatured cystine-knot protein into a buffer having an alkaline pH value comprising a solubilising non-ionic agent in a non-denaturing concentration; (d) binding to a heparin matrix of the product obtained in step (c) at a conductivity between 15 and 25 mS/cm; and (e) elution of the renatured biologically active cystine-knot protein.

The term "chaotropic agent" means, in accordance with the present invention, any substance that disturbs the three-dimensional structure of the hydrogen bonds in water. Thus, intramolecular binding forces, which are involved in the formation of spatial structures of biological molecules, are also weakened, causing the solubilization or even denaturation of biological molecules.

The term "cystine-knot protein" is, in accordance with the present invention, a protein with an unusual knot-like arrangement of three intramolecular disulfide bridges where one disulfide bond threads through a loop formed by the two other disulfide bonds. The monomers can form a dimer that are can be connected by none, one or two disulfide bonds.

The term "aggregation suppressor" in accordance with the present invention means a solute that prevents or reduces aggregation.

The term "batch" in accordance with the present invention means addition of the protein obtained in step (a) in solubilized form to the refolding buffer in a single step.

The term "pulse addition" in accordance with the present invention means addition of the protein obtained in step (a) in solubilized form to the refolding buffer in several, i.e. at least 2, preferably at least 4, more preferred at least 6, at most preferred at least 8 steps, preferably in time interval of at least 2 hours between single additions and more preferred in time intervals of about 8 hours.

As has been outlined above and in other terms the invention solves the recited technical problem by making available a technique which allows by relatively simple means to achieve a surprising effect. This was achieved by the development of an optimized purification protocol involving a renaturation procedure for cystine-knot proteins and in a preferred embodiment rhBMP-2 wherein large amounts of this protein were efficiently produced in form of inclusion bodies with recombinant bacteria, preferably *E. coli.* Optimization of the renaturation conditions for such proteins exemplified in the appended examples by rhBMP-2 with respect to pH, redox conditions, temperature, protein concentration, ionic strength and cosolvents such as low molecular weight aggregation suppressors allowed refolding at protein concentrations of 075 mg mL⁻¹ with a renaturation yield of 44%. A further increase in the concentration of rhBMP-2 was achieved by a pulsed refolding procedure (pulse refolding being a particularly preferred embodiment to be used in the method of the invention) that resulted in a final concentration of 2.1 mg mL⁻¹ rhBMP-2 with an overall renaturation yield of 33 to 38%, corresponding to 0.7 to 0.8 mg/ml renatured dimeric rhBMP-2. Thus, the protein concentration during renaturation could be increased 12 to 13 fold as compared to the previously published procedure making the *E. coli* expression system even more acceptable for commercial production.

It is preferred that the chaotropic agent in step (a) is present in a final concentration of 3 to 8 mol/L. It is also preferred that the agent in step (c) is a chaotropic agent. Also preferred is, if the protein is a BMP and preferably rhBMP, that the final concentration of said solubilizing agent in step (c) is from 3 to 6 mol/L.

Whereas the ratio of reduced and oxidized glutathione is equal or above 1:10, ratios of 2:10, 3:10, 4:10, 5:10 etc. such as 10:10, 50:10, 100:10, 200:10 or even 400:10 are feasible and work in accordance with the present invention. The broad range of concentrations of reduced glutathione and oxidized glutathione that provides excellent results is another surprising result that was found in accordance with the present invention.

It is also preferred that the refolding buffer is, prior to the refolding step, saturated with an oxygen displacing gas such as nitrogen or argon.

It is also preferred that the non-solubilizing chaotropic agent is present in step (bc) in a final concentration of 0.3 to 0.8 mol/L, for example if guanidinium hydrochloride is used as the chaotropic agent. If different chaotropic agents are employed, the final concentration may vary. The skilled artisan is in a position, based on his or her general technical knowledge, to choose a final concentration of the respective chaotropic agent that is sufficient to solubilize the protein.

In accordance with the present invention any heparin-matrix may be chosen. It is preferred in one embodiment that the heparin matrix is provided as a column and that step (d) is effected as a chromatographic step.

Any suitable bacterium can be employed for carrying the method of the invention. In a preferred embodiment of the method of the present invention said bacterium is a gram-negative bacterium.

In a more preferred embodiment of the method of the present invention said bacterium is E.coli.

A variety of chaotropic agents may be employed in step (a) including urea, perchlorate, trichloroacetate and thiocyanate. In a further preferred embodiment of the method of the present invention said chaotropic agent is guanidinium hydrochroride.

In an additional preferred embodiment of the method of the present invention said aggregation suppressor is 2-(cyclohexylamino) ethansulfonic acid (CHES) or arginine.

Renaturation yields obtained with CHES are higher than those obtained with arginine. On the other hand, arginine functions better as an aggregation suppressor.

In another preferred embodiment of the method of the present invention said cystine-knot protein is a growth factor and particular preferred selected from the group of BMPs, PDGFs, human nerve growth factors, TGF-β, VEGF, GDF-5 and biological active muteins thereof. It is also preferred that the biologically active cystine-knot protein is in the form of a dimer.

The various growth factors produced and purified in accordance with the present invention are well known in the art.
The term "mutein" in accordance with the present invention, denotes a protein that is mutated, preferably by one or more point-mutation(s), giving rise to proteins having the same, preferably improved and less preferred aggravated biological properties, in particular growth factor activities.

In still another preferred embodiment of the method of the present invention the solubilising agent used in step (c) in a non-denaturing concentration is urea.

The various chaotropic agents used in the various steps may chemically be the same agents with the proviso that where an non-ionic chaotropic agent is required, and the agent is the same in one or more of the other steps, said chaotropic agent in said other steps must also be of non-ionic nature. The concentration of chaotropic agents in the various steps may be adjusted, from step to step, by addition of chaotropic agents or by dilution. If different chaotropic agents are used in the different steps, it is preferred that the concentration recited in a specific step is the concentration of only the chaotropic agent recited in said specific step (but does not include or refer to the concentration of said other chaotropic agents that may also be present).. It is also feasible that different chaotropic agents are used to exert their activity in one and the same step.

In an additional preferred embodiment of the method of the present invention the cystine-knot protein is present in step (b) in a final concentration higher than 0.2 mg/ml. It is more preferred that the concentration is in the range of 0.3 to 0.75 mg/ml. Surprisingly, these high concentrations of protein could be treated in the method of the invention leading to a substantial purification after the heparin binding step to a purity of at least 90%, more preferably at least 95%, such as at least 98%, without a concomitant significant loss of protein: The option of starting the method of the invention with high protein concentrations and still obtaining a highly purified protein in high yields was not predictable from the combined teachings of the prior art including the paper by Vallejo et al. loc. cit. The results thus obtained allow the conclusion that the method of the invention is suitable for industrial production of cystine-knot proteins.

In a further preferred embodiment of the method the present invention the alkaline pH value in step (c) is at least 7.5 such as 8, 8.5, 9, 9.5 or 10.

Whereas temperatures of between 0°C and 20°C are feasible, in another preferred embodiment of the method of the present invention the temperature in step (b) is about 10°C.

Elution from the heparin matrix may be effected by a variety of means and using various elution buffers. In a more preferred embodiment of the method of the present invention the elution in step (e) is a salt-based elution.

In a most preferred embodiment of the method of the present invention the elution is effected with Nacl at a final concentration of at least 0.5 mol/L.

In a preferred embodiment of the method of the present invention said conductivity is between 17 and 22 mS/cm and most preferably about 20 mS/cm.

In another preferred embodiment of the method of the present invention the method further comprises (f) transferring the eluted biologically active cystine-knot protein into an acidic solution having preferably a low ionic strength; (g) optionally concentrating the protein; and (h) freeze drying or spray-drying the protein.
In this connection the "acidic solution" is intended to mean a solution having a pH value below 6 and preferably about 5.

In a more preferred embodiment of the method of the present invention the method further comprises prior to step (a) the step of (a') expressing the non-codon optimized gene encoding the cystine-knot protein in a bacterium that overexpresses rare-codon tRNAs or; (a") expressing a codon optimized gene encoding the cystine-knot protein in a bacterium that does not overexpress rare-codon tRNAs.
An appropriate bacterium is the E coli strain Rosetta® or the E.coli strain CodonPlus®, both strains are distributed by Novagen.

The term "non-codon optimized gene" in accordance with the present invention means a gene with a naturally occurring sequence which optionally comprises codons rarely occurring in the host bacterium used for expression.

The term "codon optimized gene" in accordance with the present invention means a gene wherein codons rarely occurring in the host bacteria are replaced by codons commonly found in said bacteria.

In an additional preferred embodiment of the method of the present invention the method further comprises formulating the cystine-knot protein into a pharmaceutical composition.

The pharmaceutical composition produced in accordance with the present invention may be in solid, liquid or gaseous form and may be, inter alia, in a form of (a) powder(s), (a) tablet(s), (a) solution(s) or (an) aerosol(s).

It is preferred that said pharmaceutical composition, optionally comprises a pharmaceutically acceptable carrier and/or diluent. The herein disclosed pharmaceutical composition may be partially useful for the treatment of, e.g. bone or cartilage associated diseases which may be degenerative diseases including spinal degenerative disc diseases. Said disorders comprise, but are not limited to the above recited diseases. The pharmaceutical composition of the invention is, inter alia, envisaged as inducing bone or cartilage formation. Therefore, the present invention provides for pharmaceutical compositions comprising the compounds of the invention to be used for the treatment of diseases/disorders associated with, for example, bone or cartilage related diseases or other diseases that can be treated by administration of growth factors.

Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. It is particularly preferred that said administration is carried out by injection and/or delivery. The compositions of the invention may also be administered directly to the target site, e.g., by biolistic delivery to an external or internal target site, like the brain. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Proteinaceous pharmaceutically active matter may be present in amounts between 1 ng and 10 mg/kg body weight per dose however, doses below or above this exemplary range are envisioned, especially considering the aforementioned factors. If the regimen is a continuous infusion, it should also be in the range of 1 µg to 10 mg units per kilogram of body weight per minute.

Progress can be monitored by periodic assessment. The compositions of the invention may be administered locally or systemically. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition of the invention may comprise further agents depending on the intended use of the pharmaceutical composition. Said agents may be drugs acting e.g. on the bone or cartilage. It is particularly preferred that said pharmaceutical composition comprises further agents like, e.g. interferons, interleukins or differentiation factors.

The figures show:

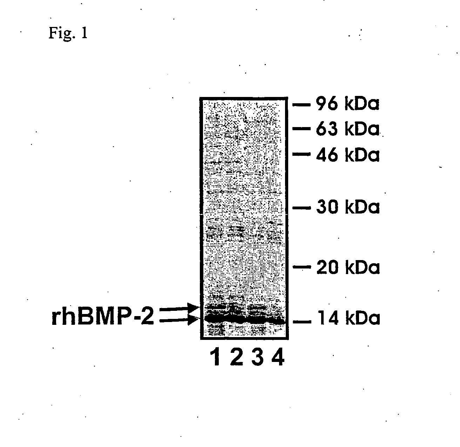

**Figure 1.** Effect of rare codon t-RNA overexpression on rhBMP-2 production. SDS-PAGE analysis of washed inclusion bodies obtained from the same biomass of *E*. *coli* strains TG1 (lanes 1 and 3) and Rosetta (lanes 2 and 4) grown in shake flask cultivation in LB (lanes 1 and 2) or defined medium (lanes 3 and 4). Induction was carried out for 4 h at 42°C. The big and small arrows point to rhBMP-2 and its higher molecular weight variant, respectively.

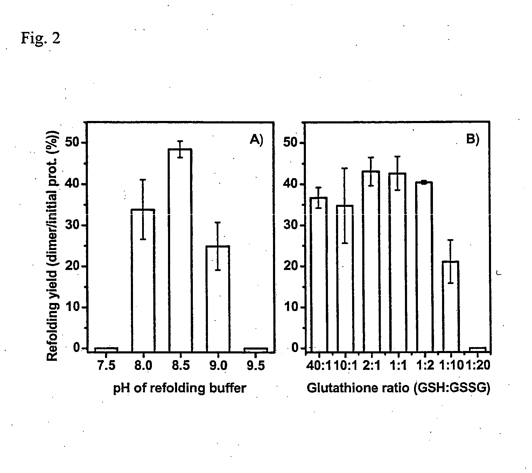

**Figure 2.** Effect of pH and redox conditions on renaturation of rhBMP-2. Renaturation was carried out at 20°C and a total concentration of 0.1 mg mL⁻¹ rhBMP-2 in standard renaturation buffer (A) with 3 mmol L⁻¹ total glutathione in a 2:1 ratio (GSH:GSSG) and the pH adjusted to the indicated values or (B) at pH 8.5 and 3 mmol L⁻¹ total glutathione at the indicated redox ratios. The renaturation yield is expressed as percentage of dimerized rhBMP-2.

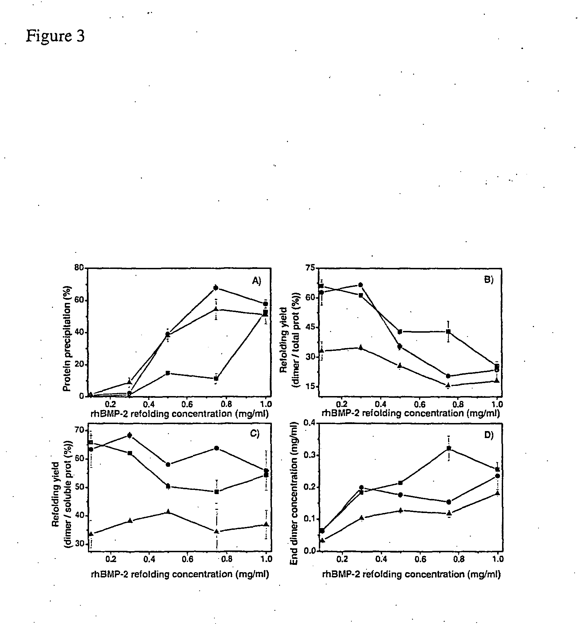

**Figure 3.** Effect of protein concentration and temperature on renaturation of rhBMP-2. Renaturation was carried out at the indicated total concentration of rhBMP-2 in standard renaturation buffer at 10°C **■,** 20°C ●, and 30°C ▲. The lower temperature limit of the renaturation experiments was given by the limited solubility of CHES at low temperatures. (A) The percentage of aggregated rhBMP-2 and (B) the renaturation yield expressed as percentage dimerized rhBMP-2. (C) The percentage dimerized rhBMP-2 in the soluble fraction of the renaturation mixture and (D) the absolute concentration of dimerized rhBMP-2.

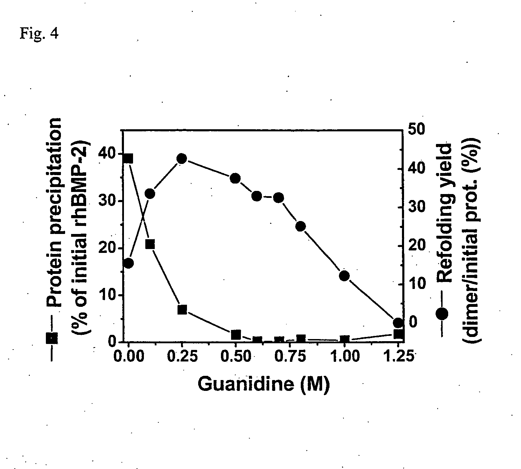

**Figure 4.** Effect of Gdn-HCl concentration on renaturation of rhBMP-2. Renaturation was carried out at 10°C and a total concentration of 0.3 mg mL⁻¹ rhBMP-2 in standard renaturation buffer. Deviating from standard buffer composition, the concentration of Gdn-HCl was as indicated. The percentage of aggregated rhBMP-2 and the renaturation yield expressed as percentage dimerized rhBMP-2 are shown.

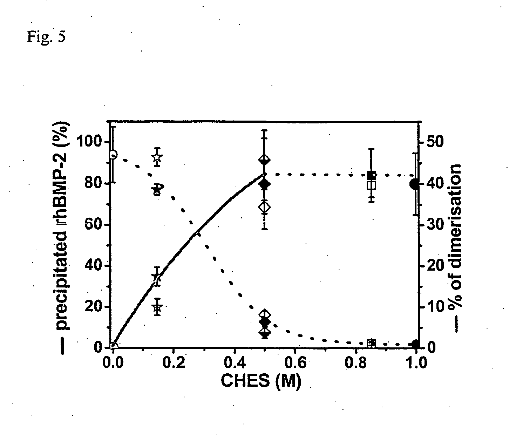

**Figure 5.** Effect of CHES and NaCl on renaturation of rhBMP-2. Renaturation was carried out at 10°C and a total concentration of 0.3 mg mL⁻¹ rhBMP-2 in standard renaturation buffer. Deviating from standard buffer composition, the renaturation buffer, containing CHES at indicated concentrations, was supplemented with NaCL as follows: 0.0 mol L⁻¹, ◆; 0.18 mol L⁻¹, 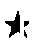 **, ■**; 0.6 mol L⁻¹, ○, ●, 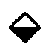 ; 1.0 mol L⁻¹, 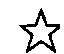 , □; 1.2 mol L⁻¹, 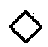 . The percentage of aggregated rhBMP-2 and the renaturation yield expressed as percentage dimerized rhBMP-2 are given.

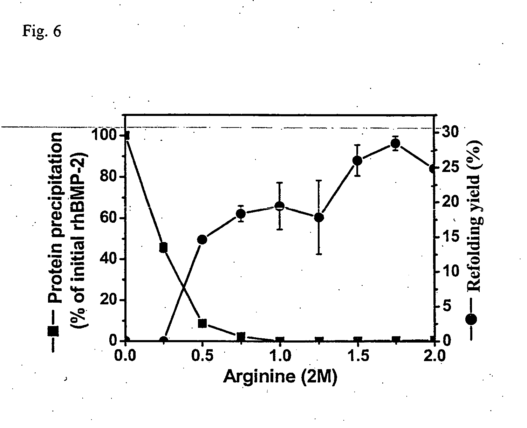

**Figure 6.** Effect of arginine on renaturation of rhBMP-2. Renaturation was carried out at 10°C and a total concentration of 0.3 mg mL⁻¹ rhBMP-2 in standard renaturation buffer. Deviating from standard buffer composition, CHES was replaced by arginine at the indicated concentrations. The percentage of aggregated rhBMP-2 and the renaturation yield expressed as percentage dimerized rhBMP-2 are shown.

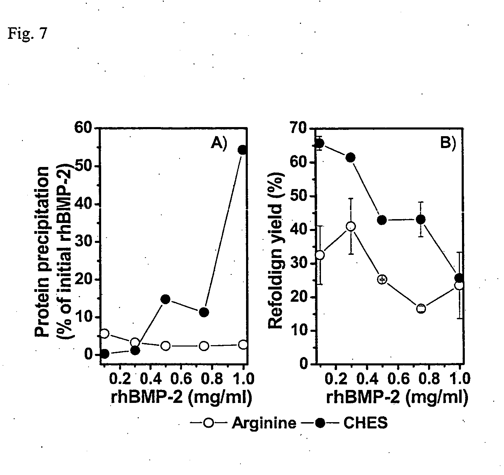

**Figure 7.** Comparison of arginine and CHES on renaturation of rhBMP-2. Renaturation was carried out at 10°C in standard renaturation buffer. Renaturation buffer contained either 0.75 mol L⁻¹ CHES (●) or 1.0 mol L⁻¹ arginine (○). (A) Suppression of aggregation of rhBMP-2 expressed as percentage of aggregated rhBMP-2 and (B) effect of aggregation suppressors on the renaturation yield expressed as percentage dimerized rhBMP-2 at different total concentrations of rhBMP-2.

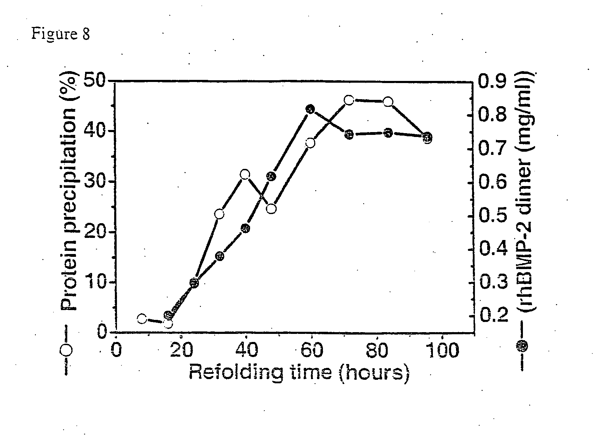

**Figure 8.** Pulse renaturation.
   Renaturation was carried out in standard renaturation buffer at 10°C. The depicted expression was started with 0.3 mg/ml rhBMP-2 and 0.5M Gnd-HCl-HCl. After each pulse addition, protein concentration was increased in 0.3 mg/ml and Gnd-HCl in 35 mM. Protein addition was done every 8 hours. Six additional pulses were added leading to a final concentration of 2.1 mg/ml of rhBMP-2. After the last pulse the refolding process was allowed to continue for 48 hours. The final conentration of dimerized active rhBMP-2 reached 0.7 to 0.8 mg/ml corresonding to a final yield of 32 to 38%. Previous to the protein addition a sample was withdrawn to assess both protein precipitation (●) and refolding yield (○). After the last pulse the refolding process was allowed to continue for 48 hours.

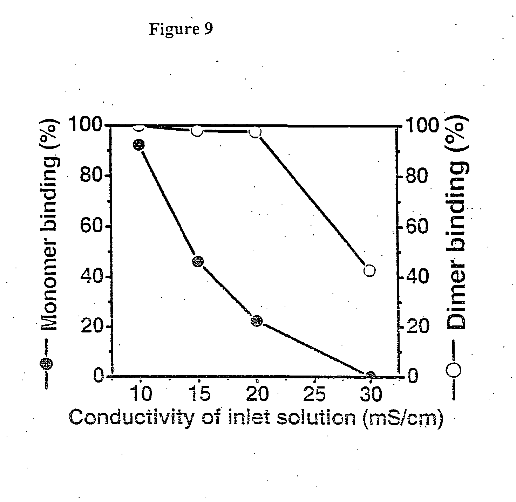

**Figure 9.** Purification of rhBMP-2 by chromatography. Effects of conductivity of the inlet solution on the selectivity of heparin for both rhBMP-2 monomer **(●)** and dimer (○). Given percentages refers to total amount of the specie at the inlet solution. Renaturation of rhBMP-2 was performed for 2 days in the standard renaturation buffer at 10°C and 0.3 mg/ml of rhBMP-2. Afterwards, protein solution was centrifuged and dialyzed against 4M urea, 20mM Tris (pH 8.0). After the dialysis process conductivity of the solution was 5mS cm⁻1 and was adjusted to the different values with 4M NaCl and applied to the heparin column. After applying the sample a 2 step NaCl gradient was used to elute rhBMP-2 monomer (0.3M) and dimer (0.5M). Amounts of the different rhBMP-2 forms in the different fractions was analyzed by SDS-PAGE.

The examples illustrate the invention.

### Example 1: Avoiding heterogeneity of rhBMP-2 by rare codon tRNA overexpression

When rhBMP-2 was produced in *E. coli* TG1:pCYTEXP3-BMP-2, a protein with a slightly higher apparent molecular weight as rhBMP-2 was also present in the inclusion body preparation (Fig. 1). N-terminal sequence analysis of rhBMP-2 and the higher molecular weight protein revealed in both cases the correct N-terminus of mature rhBMP-2 devoid of the leading bacterial methionine (GAKHKGRKRL). The rhBMP-2 gene carries several rare codons (*e.g.* 1×AGA, 1×CCC, and 1×GGA), which are known to cause translational problems such as frame-shifting or codon-hopping in *E. coli,* thereby leading sometimes to low expression levels or the appearance of undesired protein variants (#3542; #3517). These translational errors can be overcome by utilizing host strains carrying extra copies of tRNA genes cognate to these rare codons. Production of rhBMP-2 in the *E. coli* B strain Rosetta®, which supplies tRNAs for these rare codons, resulted in the complete disappearance of the higher molecular weight rhBMP-2 variant (Fig. 1). The same final specific product concentration was obtained for both strains when cells were grown on complex medium, however, a slight reduction in rhBMP-2 was observed when *E. coli* Rosetta® was grown on defined medium (Fig. 1).

### Example 2: Effect of pH and redox conditions on renaturation of rhBMP-2

pH and redox conditions are important variables for renaturation of disulfide-bonded proteins. Disulfide bond formation and disulfide reshuffling reactions are in general favored at alkaline pH and should be carried out at the highest pH where the native protein structure can be obtained (#3556). Thus, renaturation experiments of rhBMP-2 were carried out at different pH in order to establish optimum refolding conditions (Fig. 2A). No renaturation was observed up to pH 8. At more alkaline conditions (above pH 9), renaturation of rhBMP-2 was also disfavored probably due to protein instability. The obtained bell-shaped pH dependence of the refolding yield has been observed for other proteins containing disulfide bonds (#3224; #3243; #3553), however, the pH range in which rhBMP-2 renaturation can take place is exceptionally narrow, particularly when compared to other cystine-knot proteins (#3224; #3243). For renaturation of disulfide-bonded proteins, mixtures of reduced and oxidized glutathione are employed to allow disulfide-bond reshuffling until the most stable disulfide-bond structures are obtained, in general the native state of the protein. An excess of reduced glutathione of 10:1 (GSH:GSSG) has been found in some cases to result in the highest renaturation yields (#3556). Thus, optimum refolding conditions are in general obtained at specific redox conditions while more oxidizing or more reducing conditions result in low refolding yields of disulfide bonded proteins (#3218; #3248; #2857; #3553; #3555). For example, the refolding yield of the cystinee-knot family member platelet derived growth factor (PDGF) reached its maximum at a 10:1 (GSH:GSSG) redox ratio, decreasing when either more reducing or oxidizing conditions were employed (#2840). Surprisingly and in contrast to the narrow pH range, a constant renaturation yield of rhBMP-2 was obtained at a very broad range of redox conditions (Fig. 2B). Only very oxidizing conditions prohibited renaturation of rhBMP-2. Also, a variation of the total glutathione concentration from 3 to 9 mol L⁻¹ did not have any effect on the rhBMP-2 refolding yield (data not shown). Thus, in case of rhBMP-2, the pH and not the ratio of GSH:GSSG is the critical variable for optimum renaturation.

### Example 3: Effect of protein concentration and temperature on renaturation of rhBMP-2

Protein aggregation is one of the major side reactions during renaturation favored when refolding is performed at high protein concentration (#3095; #3311). Several strategies including refolding at very low protein concentration, low temperature and/or addition of aggregation suppressors (*cf*. below) are suitable for preventing or minimizing aggregation. However, low protein concentrations result in cost-intensive concentration steps and low temperatures slow down the renaturation procedure or may even result in lower renaturation yields.
For identification of improved renaturation conditions, refolding experiments were carried out in a temperature range from 10 to 30°C and rhBMP-2 concentrations up to 1.0 mg mL⁻¹ (Fig. 3). Pronounced aggregation was observed at all temperatures, when renaturation was carried out at 1.0 mg mL⁻¹ total rhBMP-2. No rhBMP-2 dimers were found in the aggregated fraction. Lower protein concentrations and lower temperatures reduced the aggregation propensity (Fig. 3A). For example, by decreasing the temperature from 30 to 10°C, the total rhBMP-2 concentration during refolding could be increased from 0.3 to 0.75 mg mL⁻¹ with less than 10 to 15% of protein lost to precipitation. Concomitant to reduced aggregation an increase in the renaturation yield was observed with declining temperatures (Fig. 3B). Remarkably, higher renaturation yields at lower temperatures were not only caused by reduced aggregation but also by a higher percentage of dimerized rhBMP-2 in the remaining soluble fraction (Fig. 3C). In summary, the highest concentrations of soluble dimer are obtained when lower refolding temperatures are used. (Fig. 3D). Best conditions for renaturation were identified at 10°C and 0.75 mg mL⁻¹ total rhBMP-2. Under these conditions, 0.3 mg mL⁻¹ dimerized rhBMP-2 was generated which represents a renaturation yield of 44%.

### Example 4: Effect of aggregation suppressors on renaturation of rhBMP-2

The standard renaturation buffer contains 0.5 mol L⁻¹ Gdn-HCl, 0.75 mol L⁻¹ CHES and 1 mol L⁻¹ Nacl (#3233). These additives are known to effect protein stability and the aggregation propensity during refolding, but also influence the subsequent purification of the dimerized growth factor. For further down-stream processing its is desirable to further increase the target protein concentration in the refolding mixture, *e.g.* by pulse renaturation *(cf.* below). Thus, the highest possible concentration of the denaturant Gdn-HCl that still permits renaturation of rhBMP-2 needs to be determined. On the other hand, for subsequent purification of the dimerized growth factor from the refolding mixture a low salt concentration is desirable (*cf*. below).
Refolding experiments in standard renaturation buffer but with varying concentrations of Gdn-HCl revealed that absence of Gdn-HCl caused severe precipitation of rhBMP-2 concomitant with a strongly reduced renaturation yield (Fig. 4). By increasing the concentration of Gdn-HCl aggregation was suppressed and refolding yields increased. Best yields were obtained at Gdn-HCl concentrations above 0.25 mol L⁻¹ and below 0.75 mol L⁻¹. Higher concentrations severely reduced the refolding yield. Thus, Gdn-HCl in conjunction with CHES are important as aggregation suppressors during refolding of rhBMP-2, but concentrations above 0.75 mol L⁻¹ Gdn-HCl should be avoided.
Furthermore, a study of the effect of both CHES and NaCl on the renaturation process was performed. To reduce the experimental effort, experiments were planned by means of 2² central composite design, with 7 central points and 2 real replications. The analysis of the obtained results indicated a key role of CHES, but not of Nacl, on the renaturation of rhBMP-2, and no interaction of the 2 variables (data not shown). Aggregation of rhBMP-2 was strongly suppressed at CHES concentrations above 0.5 mol L⁻¹, irrespective of the Nacl concentration in the renaturation buffer (Fig. 5). Also, at low concentrations of CHES the extent of precipitation was independent on the concentration of NaCl. Finally, when aggregation was suppressed by CHES concentrations above 0.5 mol L⁻¹, renaturation yields were rather independent on the concentrations of both CHES and NaCl. Thus, the concentration of CHES should be above 0.5 mol L⁻¹ while NaCl can be omitted from the renaturation buffer. This is in contrast to other reports on the renaturation of cystinee knot growth factors where the presence of Nacl in the renaturation buffer is considered indispensable (#3565).
Arginine is nowadays the most common aggregation suppressor as it has great capabilities for keeping aggregation-prone folding intermediates in solution (#3095). Refolding experiments in standard renaturation buffer where CHES was replaced by increasing concentrations of arginine revealed strong suppression of rhBMP-2 precipitation by arginine concentrations above 0.5 mol L⁻¹ concomitant to an increase in the renaturation yield up to 25-30% (Fig. 6). Renaturation yields did not decline at high concentrations of arginine as is frequently observed during the renaturation of other proteins (*e.g.* #3553) including the cystinee knot protein neurotrophin-3 (#3243). In contrast and similar to rhBMP-2, the renaturation yield of another cystine knot growth factor, human nerve growth factor, also remained unaffected by high arginine concentrations (#3224). However, renaturation yields obtained with arginine as folding additive were considerably lower than those obtained with CHES (*cf*. Figs. 4 and 5). This observation was corroborated by results obtained from renaturation experiments carried out with increasing concentrations of rhBMP-2 (Fig. 7). Although in comparison to CHES arginine was a better aggregation suppressor (Fig. 7A), renaturation yields for rhBMP-2 were always considerably lower in the presence of arginine than those obtained with CHES as additive (Fig. 7B).

### Example 5: Effect of host cell contaminants on renaturation of rhBMP-2

It has been reported that host cell inclusion body contaminants can cause reduced renaturation yields due to increased aggregation during refolding (#2564; #3218). Therefore, renaturation experiments were carried out with reduced and guanidine solubilized rhBMP-2 obtained either from refolded and purified rhBMP-2 or directly from washed inclusion bodies. There was no difference for both variants neither in the renaturation yield nor in the amount of rhBMP-2 lost to precipitation using either arginine or CHES as folding additives. Thus, with the high purity of the washed inclusion bodies (>85% of rhBMP-2) an additional purification step prior to refolding can be omitted. Moreover, the majority of the remaining proteinaceous inclusion body contaminants were present in the aggregate fraction of the refolding mixture, thereby incorporating a simultaneous purification step into the renaturation procedure.

### Example 6: Pulse refolding

Renaturation was carried out in standard renaturation buffer at 10°C. The depicted expression was started with 0.3 mg/ml rhBMP-2 and 0.5M Gnd-HCl-HCl. After each pulse addition, protein concentration was increased in 0.3 mg/ml and Gnd-HCl in 35 mM. Protein addition was done every 8 hours. Six additional pulses were added leading to a final concentration of 2.1 mg/ml of rhBMP-2. After the last pulse the refolding process was allowed to continue for 48 hours. The final conentration of dimerized active rhBMP-2 reached 0.7 to 0.8 mg/ml corresonding to a final yield of 32 to 38%. Previous to the protein addition a sample was withdrawn to assess both protein precipitation and refolding yield. After the last pulse the refolding process was allowed to continue for 48 hours.

### Example 7: Purification and storage of rhBMP-2

The dialysis step prior to heparin chromatography (#3223) could be omitted in the improved purification procedure. The absence of NaCl in the renaturation buffer decreased the conductivity from 80 to 31 mS/cm. A dilution of the renaturation mixture with 4 mol L⁻¹ urea (1:1) further decreased the conductivity to 15 mS/cm, thereby keeping the rhBMP-2 dimer in solution and allowing binding to the heparin resin while the rhBMP-2 monomer as well as the residual proteinaceous contaminants remained in the flow through. Elution of the rhBMP-2 dimer was performed at 0.5 or higher mol L⁻¹ NaCl.

For long term storage of rhBMP-2 as freeze/dried compound it is a prerequisite to remove the high concentrations of urea and Nacl. Many attempts for their removal caused immediate precipitation of rhBMP-2. The solubility of rhBMP-2 is very low in aqueous solutions at neutral pH, but it slightly increases as moderate acidic pH (#2861). By exchanging the urea/NaCl solution against 50 mmol L⁻¹ MES (pH 5.0) through ultrafiltration, concentrations of 2 mg mL⁻¹ rhBMP-2 were obtained without protein precipitation. This buffer exchange must be performed after chromatography, as elution of rhBMP-2 from the heparin column at pH 5.0 is not feasible even in the presence of high concentrations of Nacl. Freeze/drying of rhBMP-2 in e.g. 50 mmol L⁻¹ MES (pH 5.0) and subsequent rehydration did not result in any biological activity loss.

### Example 8:

### 1. Strains and plasmids

*E*. *coli* strains TG1 (DSM 6056) and Rosetta^{TM} (DE3) (Novagen, Madison, USA) were used as hosts for the temperature-inducible expression vector pCYTEXP3-BMP-2 (#3233). This expression vector carries the non-codon-optimized gene obtained from the mature hBMP-2 cDNA. The *E. coli* B strain Rosetta ^{TM} (DE3) encodes rare codon tRNAs (AUA, AGG, AGA, CUA CCC and GGA) under the control of their native promoters on a compatible plasmid.

### 2. Culture conditions, inclusion body preparation, and solubilization of inclusion body proteins

Culture conditions for cell growth on Luria Bertani (LB) or defined medium and the procedures for inclusion body preparation and solubilization of inclusion body proteins have been described before (#3233).

### 3. Renaturation experiments

To exclude any influence of host cell contaminants on the renaturation experiment, refolding attempts were always performed with purified and reduced rhBMP-2 if not indicated otherwise. Purified renatured (dimeric) rhBMP-2 was used as starting material. It was generated by standard renaturation of solubilized inclusion body proteins and subsequently purified by heparin chromatography as described previously (#3233). After heparin chromatography, the eluted rhBMP-2 dimer was concentrated to 2 to 35 mg mL⁻¹ in 6 mol L⁻¹ guanidinium hydrochlorid (Gdn-HCl), 0.1 mol L⁻¹ Tris-HCl (pH 8.5), 1 mmol L⁻¹ EDTA by ultrafiltration (5kDa vivaspin, vivascience, Binbrook, UK) and incubated overnight with 0.1 mol L⁻¹ dithiotreitol (DTT) at 20°C. Afterwards, DTT removal and buffer exchange were performed by ultrafiltration with 6 mol L⁻¹ Gdn-HCl, 50 mmol L⁻¹ MES (pH 5.0), 1 mmol L⁻¹ EDTA.

This solution containing 2 to 25 mg mL⁻¹ of unfolded and reduced rhBMP-2 was stored in aliquots at -70°C and thawed directly prior to the refolding experiment. Standard renaturation conditions were as follows: Dilution of unfolded and reduced rhBMP-2 with a final concentration of 0.1 mg mL⁻¹ rhBMP-2 in standard renaturation buffer with a final concentration of 0.5 mol L⁻¹ Gdn-HCl, 0.1 mol L⁻¹ Tris-HCl (pH 7.8), 0.75 mol L⁻¹ 2-(cyclohexylamino)ethanesulfonic acid (CHES), 1 mol L⁻¹ NaCl, 5 mmol L⁻¹ EDTA, and 3 mmol L⁻¹ total glutathione in a 2:1 ratio of glutathione reduced to glutathione oxidized (GSH:GSSG). After 48 h of incubation, the soluble and aggregated fractions of the renaturation mixture were separated by centrifugation and analyzed by gel electrophoresis under non-reducing conditions

### 5. Analytical methods

Analysis of rhBMP-2 dimer and monomer was done by electrophoresis under non-reducing conditions using precast 8-16% SDS-PAGE gels (Criterion, Biorad, Hercules, USA). Quantification of rhBMP-2 dimer and monomer on Coomassie-Brilliant-Blue-stained gels was done by densitometry (Quantiscan, Biosoft, Ferguson, USA) as described previously (#3233). Purified rhBMP-2 dimer and monomer were used as standards. The protein concentrations of the standards were determined by UV280 using a molar extinction coefficient of 18860 L mol⁻¹ cm⁻¹ calculated according to Wetlaufer (#2512).
N-terminal sequence analysis from blotted protein bands was carried out by automated Edman degradation (Protein Sequencer 470 A, Applied Biosystems, Foster City, USA) and *on-line* HPLC (12A, Applied Biosystems).
Biological activity of rhBMP-2 was analyzed by alkaline phosphatase induction in C2C12 cells (ATCC-1772) as described previously (#3592; #3233).

### Literature:

#3559
   Boden S. D.; Zdeblick T. A.; Sandhu H. S.; Heim S. E. (2000) The use of rhBMP-2 in interbody fusion cages. Spine 25: 376-38
#3561
   Bouxsein M. L.; Turek T. J.; Blake C. A.; D'Augusta D.; Li X.; Stevens M.; Seeherman H. J.; Wozney J. M. (2001) Recombinant human bone morphogenetic protein-2 accelerates healing in a rabbit ulnar osteotomy model. J. Bone Joint Surg. 83-A: 1219-1230
#918
   Buchner J.; Pastan I.; Brinkmann U. (1992) A method for increasing the yield of properly folded recombinant fusion proteins: single-chain immunotoxins from renaturation of bacterial inclusion bodies. Anal. Biochem. 205: 263-270
#2857
   De Bernardez Clark E.; Hevehan D.; Szela S.; Maachupalli-Reddy J. (1998) Oxidative renaturation of hen egg-white lysozyme. Folding vs aggregation. Biotechnol. Prog. 14: 47-54
#3095
   De Bernardez Clark E.; Schwarz E.; Rudolph R. (1999) Inhibition of aggregation side reactions during *in vitro* protein folding. Methods Enzymol. 309: 217-236
#2094
   Fischer B.; Perry B.; Summer I.; Goodenough P. (1992) A novel sequential procedure to enhance the renaturation of recombinant protein from *Escherichia coli* inclusion bodies. Protein Eng. 5: 593-596
#3223
   Gu Z.; Su Z.; Janson J.-C. (2001) Urea gradient size-exclusion chromatography enhanced the yield of lysozyme refolding. J. Chromatogr. A 918: 311-318
#3248
   Hevehan D. L.; De Bernardez Clark E. (1997) Oxidative renaturation of lysozyme at high concentrations. Biotechnol. Bioeng. 54: 221-230
#3558
   Hogan B. L .M. (1996) Bone morphogenetic proteins: multifunctional regulators of vertebrate development. Genes Dev. 1580-1594
#3565
   Honda J.; Andou H.; Mannen T.; Sugimoto S. (2000) Direct refolding of recombinant human growth differentiation factor 5 for large-scale production process. J. Biosci. Bioeng. 89: 582-589
#3595
   Israel D. I.; Nove J.; Kerns K. M.; Moutsatsos I. K.; Kaufman R. J. (1992) Expression and characterization of bone morphogenetic protein -2 in Chinese hamster ovary cells. Growth Factors 7: 139-150
#3542
   Kane J. F. (1995) Effects of rare codon clusters on high-level expression of heterologous proteins in *Escherichia coli.* Curr. Opin. Biotechnol. 6: 494-500
#3114
   Katoh S.; Katoh Y. (2000) Continous refolding of lysozyme with fed-batch addition of denatured protein solution. Process Biochem. 35: 1119-1124
#3592
   Kirsch T.; Nickel J.; Sebald W. (2000) BMP-2 antagonists emerge from alterations in the low-affinity binding epitope for receptor BMPR-II. EMBO J. 19: 3314-3324
#3591
   Kubler N. R.; Reuther J. F.; Faller G.; Kirchner T.; Ruppert R.; Sebald W. (1998) Inductive properties of recombinant human BMP-2 produced in a bacterial expression system. Int. J. Oral. Maxillofac. Surg. 27: 305-309
#3593
   Li R. H.; Wozney J. M. (2001) Delivering on the promise of bone morphogenetic proteins. Trends Biotechnol. 19: 255-265
#2564
   Maachupalli-Reddy J.; Kelley B. D.; De Bernardez Clark E. (1997) Effect of inclusion body contaminants on the oxidative renaturation of hen egg white lysozyme. Biotechnol. Prog. 13: 144-150
#3517
   McNulty D. E.; Huddleston M. J.; Claffee B.; Green.; S.; Sathe G.; Reeves R.; Patel P.; Kane J. F. (2001) Translational problems associated with the rare arginine CGG in *Escherichia coli.* Frameshifting at CGG codons in:Merten O.-W.; Mattanovich D.; Lang C.; Larsson G.; Neubauer P.; Porro D.; Postma P. W.; Teixeira de Mattos J.; Cole; J. A. (ed) Recombinant protein production with prokaryotic and eukaryotic cells. A comparative view on host physiology. Kluwer ,Dordrecht, The Netherlands ,pp151-158
#3560
   Meyer R. A.; Gruber H. E.; Howard B. A.; Tabor O. B.; Murakami T.; Kwiatkowski T. C.; Wozney J. M.; Hanley E. N. (1999) Safety of recombinant human bone morphogenic protein-2 after spinal laminectomy in the dog. Spine 24: 747-754
#2840
   Müller C.; Rinas U. (1999) Renaturation of heterodimeric platelet-derived growth factor from inclusion bodies of recombinant *Escherichia coli* using size exclusion chromatography. J. Chromatogr. A 855: 203-213
#3224
   Rattenholl A.; Lilie H.; Grossmann A.; Stern A.; Schwarz E.; Rudolph R. (2001) The pro-sequence facilitates folding of human nerve growth factor from *Escherichia coli* inclusion bodies. Eur. J. Biochem. 268: 3296-3303
#3556
   Rudolph R.; Böhm G.; Lilie H.; Jaenicke R. (1997) Folding proteins In:Creighton T. E. (ed) Protein function. A practical approach. IRL Press ,Oxford New York Tokio ,pp57-99
#2861
   Ruppert R.; Hoffmann E.; Sebald W. (1996) Human bone morphogenetic protein 2 contains a heparin-binding site which modifies its biological activity. Eur. J. Biochem. 237: 295-302
#2859
   Scheufler C.; Sebald W.; Hülsmeyer M. (1999) Crystal structure of human bone morphogenetic protein-2 at 2.7 A resolution. J. Mol. Biol. 287: 103-115
#3553
   Sijwali P. S.; . Brinen L. S.; Rosenthal; P. J. (2001) Systematic optimization of expression and refolding of the *Plasmodium falciparum* cysteine protease Falcipain-2. Protein Expr. Purif. 22: 128-134
#3243
   Suenaga M.; Ohmae H.; Tsuji S.; Itoh; T. Nishimura O. (1998) Renaturation of recombinant human neurotrophin-3 from inclusion bodies using an aggreration supressor. Biotechnol. Appl. Biochem. 28: 119-124
#2073
   Terashima M.; Suzuki K.; Katoh S. (1996) Effective refolding of fully reduced lysozyme with a flow-type reactor. Process Biochem. 31: 341-345
#3218
   Tran-Moseman A.; Schauer N.; De Bernardez Clark E. (1999) Renaturation of *Escherichia coli*-derived recombinant human macrophage colony-stimulating factor. Protein Expr. Purif. 16: 181-189
#3562
   Valentin-Opran A.; Wozney J.; Csimma C.; Lilly L.; Riedel G. E. (2002) Clinical evaluation of recombinant human bone morphogenetic protein-2. Clin Orthop. 395: 110-120
#3311
   Vallejo L. F.; Rinas U. (2002) Strategies for refolding inclusion body proteins In:Villaverde A. (ed) Protein production in bacterial cell factories. Research Signpost ,Trivandrum, Kerala, India ,pp59-71
#3233
   Vallejo L. F.; Brokelmann M.; Marten S.; Trappe S.; Cabrera-Crespo J.; Hoffmann A.; Gross G.; Weich H. A.; Rinas U. (2002) Renaturation and purification of bone morphogenetic protein-2 produced as inclusion bodies in high-cell-density cultures of recombinant *Escherichia coli.* J. Biotechnol. 94: 185-194
#3085
   Vicik S. M. (1999) Methods of refolding proteins by use of zwitterionic low molecular weight agents. Patent Application No. WO 99/18196.
#3594
   Wang E. A.; Rosen V.; D'Alessandro J. S.; Bauduy M.; Cordes P.; Harada T.; Israel D. I.; Hewick R. M.; Kerns K. M.; LaPan P.; Luxenberg D. P.; McQuaid D.; Moutsatsos I. K.; Nove J.; Wozney J. M. (1990) Recombinant human bone morphogenetic protein induces bone formation. Proc. Natl. Acad. Sci. USA 87: 2220-2224
#2512
   Wetlaufer D. B. (1962) Ultraviolet spectra of proteins and amino acids. Adv. Prot. Chem. 17: 303-390
#3555
   Winter J.; Lilie H.; Rudolph R. (2002) Renaturation of human proinsulin - a study on refolding and conversion to insulin. Anal. Biochem. 310: 148-155

## Claims

1. A method of producing a biologically active recombinant cystine-knot protein comprising
(a) solubilisation of inclusion bodies comprising said cystine-knot protein produced in a bacterium in the presence of a chaotropic agent;
(b) renaturation of the solubilized cystine-knot protein in batch or by pulse addition of said solubilized cystine-knot protein to a refolding buffer essentially devoid of NaCl having a pH value of 7.5 to 9.5 comprising
(ba) an aggregation suppressor in a final concentration of at least 0.5 mol/L;
(bb) a mixture of reduced and oxidized glutathione wherein the ratio of reduced to oxidized glutathione is equal or above 1:10; and
(bc) a solubilizing chaotropic agent in a non-denaturing concentration, wherein said renaturation is carried out at a temperature between 0°C and 20°C;
(c) dialysis of the renatured cystine-knot protein against or dilution of the renatured cystine-knot protein into a buffer having an alkaline pH value comprising a solubilising non-ionic agent in a non-denaturing concentration;
(d) binding to a heparin matrix of the product obtained in step (c) at a conductivity between 15 and 25 mS/cm; and
(e) elution of the renatured biologically active cystine-knot protein.

2. The method of claim 1 wherein said bacterium is a gram-negative bacterium.

3. The method of claim 2 wherein said bacterium is E.coli.

4. The method of any one of claims 1 to 3 wherein said chaotropic agent is guanidinium hydrochroride.

5. The method of any one of claims 1 to 4 wherein said aggregation suppressor is 2-(cyclohexylamino)ethansulfonic acid (CHES) or arginine.

6. The method of any one of claims 1 to 5, wherein said cystine-knot protein is a growth factor.

7. The method of claim 6 wherein said cystine-knot protein is selected from the group of BMPs, PDGFs, human nerve growth factors, TGF-ß, VEGF, GDF-5 and biological active muteins thereof.

8. The method of any one of claims 1 to 7 wherein the non-ionic solubilizing agent in step (c) is a non-denaturing concentration is urea.

9. The method of any one of claims 1 to 8 wherein the cystine-knot protein is present in step (b) in renatured form in a final concentration higher than 0.2 mg/ml.

10. The method of any one of claims 1 to 9 wherein the alkaline pH value in step (c) is at least 8.

11. The method of any one of claims 1 to 10 wherein the temperature in step (b) is about 10°C.

12. The method of any one of claims 1 to 11 wherein the elution in step (e) is a salt-based elution.

13. The method of claim 12 wherein the elution is effected with NaCl at a final concentration of at least 0.5 mol/L.

14. The method of any one of claims 1 to 13 wherein said conductivity is about 20 mS/cm.

15. The method of any one of claims 1 to 14 further comprising
(f) transferring the eluted biologically active cystine-knot protein into an acidic solution;
(g) optionally concentrating the protein; and
(h) freeze drying or spray-drying the protein.

16. The method of any one of claims 1 to 15 further comprising, prior to step (a), the step of (a') expressing the non-codon optimized gene encoding the cystine-knot protein in a bacterium that overexpresses rare-codon tRNAs or; (a") expressing a codon optimized gene encoding the cystine-knot protein in a bacterium that does not overexpress rare-codon tRNAs.

17. The method of any one of claims 1 to 16 further comprising formulating the cystine-knot protein into a pharmaceutical composition.
